(19) Europäisches Patentamt
European Patent Office
Office européen des brevets

(11) Veröffentlichungsnummer: **0 250 961 B1**

(12) **EUROPÄISCHE PATENTSCHRIFT**

(45) Veröffentlichungstag der Patentschrift: **22.07.92**

(51) Int. Cl.$^5$: **C07D 239/26**, C07C 69/66, C07C 69/90, C09K 19/34, C09K 19/20, C09K 19/58

(21) Anmeldenummer: **87108377.0**

(22) Anmeldetag: **10.06.87**

Die Akte enthält technische Angaben, die nach dem Eingang der Anmeldung eingereicht wurden und die nicht in dieser Patentschrift enthalten sind.

# (54) Chirale Ester aus alpha-substituierten Phenylalkansäuren und mesogenen Hydroxylverbindungen und ihre Verwendung als Dotierstoff in Flüssigkristall-Phasen.

(30) Priorität: **14.06.86 DE 3620049**

(43) Veröffentlichungstag der Anmeldung:
**07.01.88 Patentblatt 88/01**

(45) Bekanntmachung des Hinweises auf die Patenterteilung:
**22.07.92 Patentblatt 92/30**

(84) Benannte Vertragsstaaten:
**AT BE CH DE ES FR GB IT LI NL SE**

(56) Entgegenhaltungen:
**EP-A- 0 159 872**
**EP-A- 0 217 240**

**MOLECULAR CRYSTALS AND LIOUID CRYSTALS, Band 148, 1987, Papers Presented at the 11th International Liquid Crystal Conference, Berkeley, California 30. Juni - 4. July 1986, Seiten 29-43, Gordon and Breach Science Publishers, New York, US; CH. BAHR et al.: "Ferroelectric liquid crystals: Properties of binary mixtures and pure compounds with high spontaneous polarisation"**

(73) Patentinhaber: **HOECHST AKTIENGESELLSCHAFT**
**Postfach 80 03 20**
**W-6230 Frankfurt am Main 80(DE)**

(72) Erfinder: **Heppke, Gerd, Prof. Dr.**
**Johann-Georg-Strasse 3**
**W-1000 Berlin 31(DE)**
Erfinder: **Scherowsky, Günter, Prof. Dr.**
**Winklerstrasse 18B**
**W-1000 Berlin 33(DE)**
Erfinder: **Bahr, Christian**
**Hohenzollerndamm 189**
**W-1000 Berlin 31(DE)**
Erfinder: **Lehmann, Lutz**
**Bondickstrasse 59a**
**W-1000 Berlin 28(DE)**

EP 0 250 961 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Kennlinien der in Flüssigkristall-Displays verwendeten elektro-optischen Effekte verändern sich im allgemeinen mit der Temperatur. Insbesondere bei einer Ansteuerung im Multiplexbereich ergeben sich daraus Schwierigkeiten, die zu einer unerwünschten Einschränkung des Arbeitstemperaturbereiches führen können. Bei verschiedenen elektrooptischen Effekten kann durch Zusatz chiraler Verbindungen zum nematischen Flüssigkristall über die Temperaturfunktion der Ganghöhe der dadurch induzierten cholesterischen Helixstruktur die Temperaturabhängigkeit der elektrooptischen Kennlinien vorteilhaft beeinflußt werden, so beim cholesterisch-nematischen Phasenumwandlungseffekt, der TN ("twisted nematic")-Zelle und dem kürzlich vorgestellten SBE ("supertwisted birefringence effect") und dem STN ("super twisted nematic")-Effekt.

Neben den nematischen und cholesterischen Flüssigkristallen haben seit einigen Jahren in zunehmendem Maße auch für Praxisanwendungen geneigt("tilted")-smektische Flüssigkristall-Phasen an Bedeutung gewonnen. Wenn solchen geneigt-smektischen Phasen, insbesondere smektisch C ($S_c$ oder SmC)Phasen, geeignete Dotierstoffe zugesetzt werden, die eine sogenannte spontane Polarisation ($P_s$) zeigen, so können die genannten Phasen in eine ferroelektrische Flüssigkristall-Phase umgewandelt werden (Benennung von $P_s$ in $nC\cdot cm^{-2}$), siehe dazu beispielsweise Lagerwall et al. im Aufsatz "Ferroelectric Liquid Crystals for Displays" (Ferroelektrische Flüssigkristalle für Anzeigenelemente), SID Symposium, October Meeting, 1985, San Diego (USA).

In der EP-A 0 159 872 werden Verbindungen u.a. der nachstehenden allgemeinen Formal beschrieben, die als Komponente in Flüssigkristall-Systemen geeignet sein sollen, wobei sie hohe Werte für die spontane Polarisation zeigen:

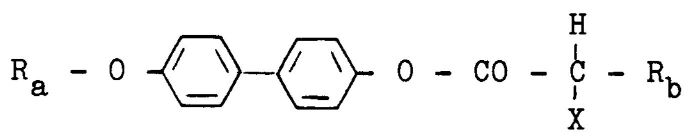

Die Substituenten sollen dabei folgende Bedeutung haben:
$R_a$ = $(C_1\text{-}C_{18})$Alkyl, X = F, Cl oder Br und $R_b$ = Benzyl oder Phenyl.

Aufgabe der vorliegenden Erfindung ist es, Verbindungen aufzuzeigen, die bei hohen Werten für die spontane Polarisation $P_s$ Strukturelemente aufweisen, die sie auch mit anderen Komponenten in Flüssigkristall-Systemen "verträglich" (d. h. mischbar) machen, da Strukturelemente im mesogenen Teil und/oder chiralen Teil der Moleküle oftmals für eine gute "Verträglichkeit" mit den anderen Mischungskomponenten in Flüssigkristall-Systemen verantwortlich sind.

Die Erfindung geht aus von den bekannten chiralen Estern aus α-substituierten Phenylalkansäuren und mesogenen Hydroxylverbindungen. Die erfindungsgemäßen Ester sind dann dadurch gekennzeichnet, daß in der allgemeinen Formel (I)

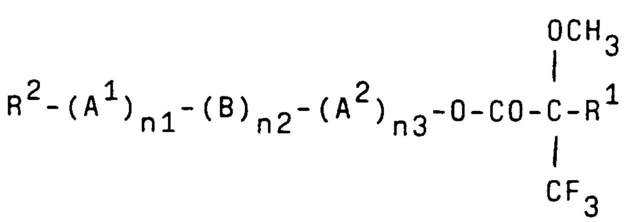 (I)

die Symbole folgende Bedeutung haben:

| | |
|---|---|
| $R^2$ | ist ein geradkettiger $(C_4\text{-}C_{10})$-Alkylrest, in dem eine $CH_2$-Gruppe durch O oder S ersetzt sein kann, |
| $A^1$ und $A^2$ | sind unabhängig voneinander 1,4-Phenylen 1,4-Cyclohexylen oder Pyrimidin-2,5-diyl, |
| B | ist CO-O oder O-CO, |
| n1 | ist 1, |
| n2 | ist 0 oder 1, |
| n3 | ist 1 oder 2 und |
| $R^1$ | ist Phenyl. |

Die genannten Verbindungen sind chirale α-mono oder disubstituierte (d.h. in 2-Stellung einer chiralen Phenylalkansäure substituierte) Phenylessig- oder Phenylpropionsäureester von Phenolen (oder vergleichbaren heterocyclischen Verbindungen) oder Cycloalkanolen.

Eine weitere Lösung der gestellten Aufgabe ist eine verdrillbare Flüssigkristall-Phase mit einem Gehalt an mindestens einer chiralen Verbindung, die als chirale Verbindung mindestens eine Verbindung der allgemeinen Formel (I) enthält. Unter dem Begriff "verdrillbare Flüssigkristall-Phase" sind nematische, cholesterische oder geneigt("tilted")-smektische, insbesondere SmC-Phasen zu verstehen.

Die verdrillbaren Flüssigkristall-Phasen bestehen aus 2 bis 20, vorzugsweise 2 bis 15 Komponenten, darunter mindestens einer der erfindungsgemäß beanspruchten chiralen Verbindungen. Die anderen Bestandteile werden vorzugsweise ausgewählt aus den bekannten Verbindungen mit nematischen, cholesterischen und/oder geneigt-smektischen Phasen, dazu gehören beispielsweise Schiffsche Basen, Biphenyle, Terphenyle, Phenylcyclohexane, Cyclohexylbiphenyle, Pyrimidine, Zimtsäureester, Cholesterinester, verschieden überbrückte, terminal-polare mehrkernige Ester von p-Alkylbenzoesäuren. Im allgemeinen liegen die im Handel erhältlichen Flüssigkristall-Phasen bereits vor der Zugabe der chiralen Verbindung(en) als Gemische verschiedenster Komponenten vor, von denen mindestens eine mesogen ist, d.h. als Verbindung, in derivatisierter Form oder im Gemisch mit bestimmten Cokomponenten eine Flüssigkristall-Phase zeigt [ = mindestens eine enantiotrope (Klärtemperatur> Schmelztemperatur) oder monotrope (Klärtemperatur< Schmelztemperatur) Mesophasenbildung erwarten läßt].

Unter den Verbindungen der allgemeinen Formel (I) sind die bevorzugt, bei denen die Symbole folgende Bedeutung haben:

$R^2$ = geradkettiges ($C_4$-$C_{10}$)Alkyl, wobei eine $CH_2$-Gruppe durch ein O- oder S-Atom ersetzt sein kann, $A^1$,$A^2$ = unabhängig voneinander unsubstituiertes 1,4-Phenylen, 1,4-Cyclohexylen oder Pyrimidin-2,5-diyl, B = CO-O oder O-CO, n1 = 1, n2 = 0 oder 1 und n3 = 1 oder 2, Y = $CF_3$, und Z = $OCH_3$ oder Y = $OCH_3$ und Z = H, und $R^1$ = unsubstituiertes Phenyl.

Die Herstellung der erfindungsgemäßen Verbindungen erfolgt zweckmäßig durch Umsetzung von mesogenen Verbindungen der Formel (III) oder (III′)

MOH (III)

$(MO)_m$Me (III′)

in denen MO die oben angegebene Bedeutung hat, Me für ein Erdalkali- oder, vorzugsweise ein Alkalimetall steht und m = 1 (Me = Alkalimetall) oder m = 2 (Me = Erdalkalimetall) ist, mit Verbindungen der Formel (IV)

$$X^f\text{-CO-}\underset{Y}{\overset{Z}{C}}\text{-}R^1 \qquad (IV)$$

in der $X^f$ eine OH-Gruppe oder Halogen, vorzugsweise Chlor darstellt, und Z und Y die oben angegebene Bedeutung haben.

Vorzugsweise werden zur Herstellung der Verbindungen mesogene Hydroxylverbindungen (III) und Säurechloride (IV; $X^f$ = Cl) eingesetzt, wobei die Umsetzung in Gegenwart von Säurefängern wie Aminen, beispielsweise Pyridin oder Triethylamin, oder von Alkali- oder Erdalkali(hydrogen)carbonaten, im allgemeinen bei Temperaturen zwischen -40 °C und +70 °C erfolgt. Es ist aber auch möglich, die mesogene Hydroxylverbindung (III) mit der Carbonsäure (IV, $X^f$ = OH) selbst umzusetzen, und zwar in Gegenwart von Brönstedt-oder Lewis-Säuren, ggf. in Gegenwart wasserbindender Mittel, oder unter Inhilfenahme von Kondensations-Reagentien wie N,N′ -Carbonyldiimidazol, Dicyclohexylcarbodiimid oder Azodicarbonsäureester/Triphenylphosphin.

Zu den Verbindungen (I) gelangt man schließlich auch durch Umsetzung der Alkali- oder Erdalkalisalze der mesogenen Hydroxylverbindung (III′) mit dem Carbonsäurehalogenid (IV, $X^f$ = Halogen).

Das erhaltene Rohprodukt (I) kann in an sich bekannter Weise gereinigt werden, beispielsweise durch Umkristallisieren oder durch Säulenchromatographie.

Von der oder den erfindungsgemäßen Verbindung(en) enthalten die Flüssigkristall-Phasen im allgemeinen, 0,01 bis 70 Gew.-%, insbesondere 0,05 bis 50 Gew.-%.

Die erfindungsgemäßen Verbindungen sind insbesondere als Dotierstoffe für geneigt-smektische Flüssigkristall-Phasen geeignet, da sie diese in ferroelektrische Flüssigkristall-Phasen umwandeln.

Beispiele 1 bis 4

Die Phenol- oder Cycloalkanolkomponente wird in Pyridin gelöst und tropfenweise mit einer äquimolaren Menge des (-)-2-Methoxy-2-trifluormethyl-2-phenyl-ethanoylchlorid versetzt. Unter Feuchtigkeitsausschluß wird während 60 min. auf 65 °C erwärmt und anschließend während 12 h bei Raumtemperatur gerührt. Danach wird das Gemisch in Eiswasser gegossen, es wird mit konz. Salzsäure angesäuert, mehrmals ausgeethert und die vereinigten Etherphasen mit wäßriger $NaHCO_3$-Lösung gewaschen. Nach dem Trocknen der Etherphasen uber $MgSO_4$, Einengen auf ein kleineres Volumen und Übersättigung der Phasen mit Petrolether kristallisieren die nachstehenden Produkte im Kühlschrank aus:

(1) 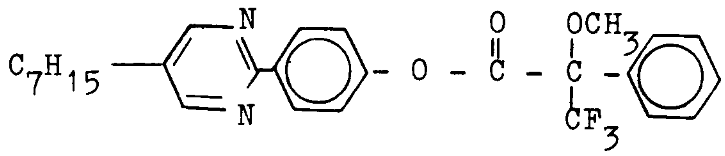

(2) 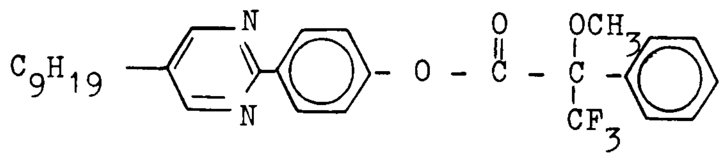

(3) 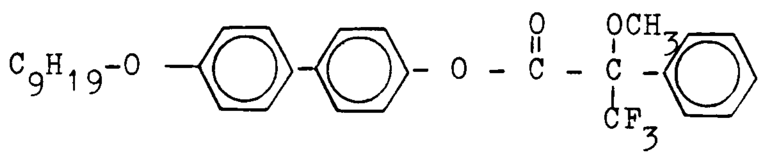

(4) 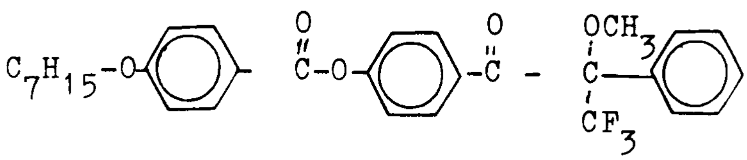

Beispiele 5 und 6

Es wird R-(-)-2-Methoxy-2-phenyl-ethansäure in Benzol mit $SOCl_2$ während 1 h unter Rückfluß erhitzt und anschließend im Vakuum eingeengt und in absolutem Benzol aufgenommen. Dazu wird eine Lösung der Phenol- oder Cycloalkanolkomponente in Benzol zugegeben und das Reaktionsgemisch mit Pyridin versetzt. Es wird während 12 h gerührt, in Wasser gegossen, mit Ether ausgeschüttelt und die organische Phase nacheinander mit wäßriger $K_2CO_3$-Lösung, wäßriger gesättigter NaCl-Lösung, wäßriger 3N HCl-Lösung und wäßriger gesättiger NaCl-Lösung behandelt. Danach wird getrocknet (über $MgSO_4$), eingeengt, und das Reaktionsprodukt durch Säulenchromatographie an Kieselgel mit Methylenchlorid als Laufmittel gereinigt.

(5) 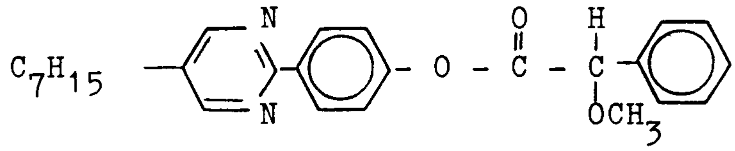

(6) 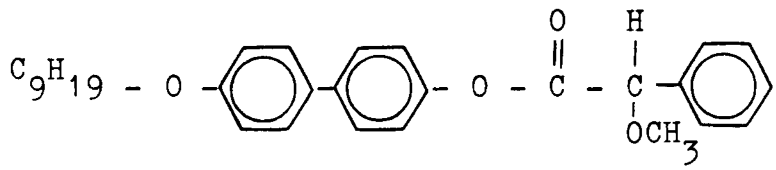

| Beispiel | Schmelzpunkt (°C) | $[\alpha]_D$ in $CHCl_3$ | $P_S$ $(nC \cdot cm^{-2})$/ bei °C |
|---|---|---|---|
| 1 | 51 | + 55° | - |
| 2 | 40,5 | + 54° | 5,6 / 75 |
| 3 | 42 | - | 9,6 / 76 |
| 4 | 39 | - | 6,6 / 70 |
| 5 | 35 | - 71° | - |
| 6 | 68 | - 70° | 14,0 / 70 |

Die spontane Polarisation ($P_s$) wird in der SmC-Phase der handelsüblichen Verbindung "HEPTOAB" (Hersteller z.B. Frinton - USA) mit den Kenndaten "K 74,4 SmC 95,4 N 124,2 I" bestimmt, wobei vom jeweiligen Dotierstoff 10 mol% zugesetzt werden.

HEPTOAB 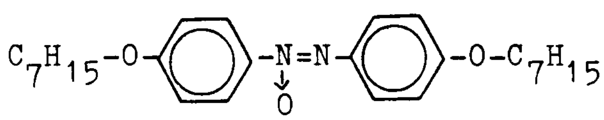

Beispiel 7

Die erfindungsgemäßen Verbindungen eignen sich auch sehr gut zur Induzierung einer cholesterischen Helixstruktur in einer nematischen Flüssigkristallphase, um so, z.B. wie anfangs beschrieben, die Temperaturabhängigkeit der elektrooptischen Kennlinie von Flüssigkristalldisplays positiv zu beeinflussen.

Zur Bestimmung des Verdrillungsvermögens wurde die Verbindung (3) zu einer handelsüblichen nematischen Weitbereichsmischung - "RO-TN 404" der Hoffmann-La Roche Aktiengesellschaft (Basel/Schweiz) - mit einem Klärpunkt von 104° C zugesetzt.

Die Werte für das Verdrillungsvermögen in der nematischen Flüssigkristallphase in µm.Gew.-% (= p•c) betragen:

| Meßtemperatur [°C] | 10 | 20 | 30 | 40 | 50 | 60 |
|---|---|---|---|---|---|---|
| p•c [µm•Gew.-%] | -12,3 | -12,3 | -12,4 | -12,5 | -12,5 | -12,6 |

**Patentansprüche**

**Patentansprüche für folgende Vertragsstaaten : BE, CH, DE, FR, GB, IT, LI, LU, NL, SE**

**1.** Chiraler Ester der Formel I,

$$R^2-(A^1)_{n1}-(B)_{n2}-(A^2)_{n3}-O-CO-\underset{\displaystyle CF_3}{\overset{\displaystyle OCH_3}{C}}-R^1 \qquad (I)$$

in der

$R^2$ einen geradkettigen ($C_4$-$C_{10}$)-Alkylrest bedeutet, in dem eine $CH_2$-Gruppe durch O oder S ersetzt sein kann,
$A^1$ und $A^2$ unabhängig voneinander 1,4-Phenylen 1,4-Cyclohexylen oder Pyrimidin-2,5-diyl sind,
B CO-O oder O-CO,
n1 1,
n2 O oder 1,
n3 1 oder 2 und
$R^1$ Phenyl bedeuten.

**2.** Verdrillbare Flüssigkristall-Phase mit einem Gehalt an mindestens einer chiralen Verbindung, dadurch gekennzeichnet, daß sie mindestens eine chirale Verbindung der allgemeinen Formel (I) nach Anspruch 1 enthält.

**3.** Flüssigkristall-Anzeigeelement enthaltend eine Flüssigkristall-Phase nach Anspruch 3.

**4.** Verwendung einer chiralen Verbindung gemäß der allgemeinen Formel (I) nach Anspruch 1 zur Umwandlung einer geneigt-smektischen in eine ferroelektrische Flüssigkristall-Phase.

**5.** Verfahren zur Umwandlung einer geneigt-smektischen in eine ferroelektrische Flüssigkristall-Phase durch Zusatz von mindestens einer chiralen Verbindung, dadurch gekennzeichnet, daß man der Flüssigkristall-Phase mindestens eine Verbindung der allgemeinen Formel (I) nach Anspruch 1 zusetzt.

**Patentansprüche für folgenden Vertragsstaat : AT**

**1.** Verfahren zum Herstellen von chiralen Estern der Formel (I)

$$R^2-(A^1)_{n1}-(B)_{n2}-(A^2)_{n3}-O-CO-\underset{\displaystyle CF_3}{\overset{\displaystyle OCH_3}{C}}-R^1 \qquad (I)$$

die Symbole folgende Bedeutung haben:

$R^2$ ist ein geradkettiger ($C_4$-$C_{10}$)-Alkylrest, in dem eine $CH_2$-Gruppe durch O oder S ersetzt sein kann,
$A^1$ und $A^2$ sind unabhängig voneinander 1,4-Phenylen 1,4-Cyclohexylen oder Pyrimidin-2,5-diyl,
B ist CO-O oder O-CO,
n1 ist 1,
n2 ist 0 oder 1,
n3 ist 1 oder 2 und
$R^1$ ist Phenyl,

dadurch gekennzeichnet, daß man mesogene Verbindungen der Formel (III) oder (III')

MOH (III)
$(MO)_mMe$ (III')

in denen MO die oben angegebene Bedeutung hat, Me für ein Erdalkali- oder Alkalimetall steht und m = 1 für Me = Alkalimetall und m = 2 für Me = Erdalkalimetall ist, umsetzt mit Verbindungen der Formel (IV)

$$X^{f}-CO-\underset{Y}{\overset{Z}{C}}-R^{1} \qquad (IV)$$

in der $X^f$ eine OH-Gruppe oder Halogen darstellt und $Z = OCH_3$, $Y = OCF_3$ und $R^1$ die oben angegebene Bedeutung haben.

**2.** Verfahren nach Anspruch 1, dadurch gekennzeichnet, daß man mesogene Verbindungen der Formel (III) oder (III'), in denen
$R^2$ = geradkettiges ($C_4$-$C_{10}$)Alkyl, wobei eine $CH_2$-Gruppe durch Sauerstoff oder Schwefel ersetzt sein kann,
$A^1$, $A^2$ = unabhängig voneinander umsubstituiertes 1,4-Phenylen, 1,4-Cyclohexylen oder Pyrimidin-2,5-diyl, B = CO-O oder O-CO,
n1 = 1,
n2 = null oder 1 und
n3 = 1 oder 2,
umsetzt mit einer Verbindung der Formel (IV), in der
Y = $CF_3$ und Z = $OCH_3$ oder
Y = $OCH_3$ und Z = H und
$R^1$ = unsubstituiertes Phenyl.

**3.** Verdrillbare Flüssigkristall-Phase mit einem Gehalt an mindestens einer chiralen Verbindung, dadurch gekennzeichnet, daß sie mindestens eine chirale Verbindung der allgemeinen Formel (I) nach Anspruch 1 enthält.

**4.** Flüssigkristall-Anzeigeelement enthaltend eine Flüssigkristall-Phase nach Anspruch 3.

**5.** Verwendung einer chiralen Verbindung gemäß der allgemeinen Formel (I) nach Anspruch 1 zur Umwandlung einer geneigt-smektischen in eine ferroelektrische Flüssigkristall-Phase.

**6.** Verfahren zur Umwandlung einer geneigt-smektischen in eine ferroelektrische Flüssigkristall-Phase durch Zusatz von mindestens einer chiralen Verbindung, dadurch gekennzeichnet, daß man der Flüssigkristall-Phase mindestens eine Verbindung der allgemeinen Formel (I) nach Anspruch 1 zusetzt.

## Patentansprüche für folgenden Vertragsstaat : ES

**1.** Verfahren zum Herstellen von chiralen Estern der Formel (I)

$$R^{2}-(A^{1})_{n1}-(B)_{n2}-(A^{2})_{n3}-O-CO-\underset{CF_3}{\overset{OCH_3}{C}}-R^{1} \qquad (I)$$

die Symbole folgende Bedeutung haben:
$R^2$ ist ein geradkettiger ($C_4$-$C_{10}$)-Alkylrest, in dem eine $CH_2$-Gruppe durch O oder S

ersetzt sein kann,

| | |
|---|---|
| $A^1$ und $A^2$ | sind unabhängig voneinander 1,4-Phenylen 1,4-Cyclohexylen oder Pyrimidin-2,5-diyl, |
| B | ist CO-O oder O-CO, |
| n1 | ist 1, |
| n2 | ist 0 oder 1, |
| n3 | ist 1 oder 2 und |
| $R^1$ | ist Phenyl, |

dadurch gekennzeichnet, dab man mesogene Verbindungen der Formel (III) oder (III')

MOH (III)
$(MO)_m$ (III')

in denen MO die oben angegebene Bedeutung hat, Me für ein Erdalkali- oder Alkalimetall steht und m = 1 für Me = Alkalimetall und m = 2 für Me = Erdalkalimetall ist, umsetzt mit Verbindungen der Formel (IV)

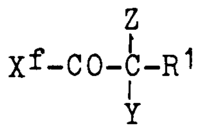
(IV)

in der $X^f$ eine OH-Gruppe oder Halogen darstellt und $Z = OCH_3$, $Y = OCF_3$ und $R^1$ die oben angegebene Bedeutung haben.

**2.** Verfahren nach Anspruch 1, dadurch gekennzeichnet, daß man mesogene Verbindungen der Formel (III) oder (III'), in denen

| | |
|---|---|
| $R^2$ = | geradkettiges $(C_4\text{-}C_{10})$Alkyl, wobei eine $CH_2$-Gruppe durch Sauerstoff oder Schwefel ersetzt sein kann, |
| $A^1$, $A^2$ = | unabhängig voneinander umsubstituiertes 1,4-Phenylen, 1,4-Cyclohexylen oder Pyrimidin-2,5-diyl, |
| B = | CO-O oder O-CO, |
| n1 = | 1, |
| n2 = | null oder 1 und |
| n3 = | 1 oder 2, |

umsetzt mit einer Verbindung der Formel (IV), in der

| | |
|---|---|
| Y = | $CF_3$ und Z = $OCH_3$ oder |
| Y = | $OCH_3$ und Z = H und |
| $R^1$ = | unsubstituiertes Phenyl. |

**3.** Verfahren zur Umwandlung einer geneigt-smektischen in eine ferroelektrische Flüssigkristall-Phase durch Zusatz von mindestens einer chiralen Verbindung, dadurch gekennzeichnet, daß man der Flüssigkristall-Phase mindestens eine Verbindung der allgemeinen Formel (I) nach Anspruch 1 zusetzt.

**4.** Flüssigkristall-Anzeigeelement enthaltend eine Flüssigkristall-Phase nach Anspruch 3.

**Claims**
**Claims for the following Contracting States : BE, CH, DE, FR, GB, IT, LI, LU, NL, SE**

**1.** A chiral ester of the formula I

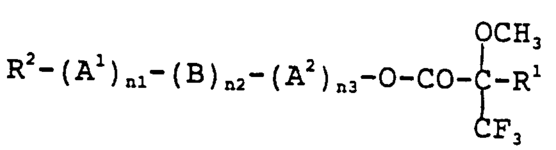
(I)

in which

| | |
|---|---|
| $R^2$ | is a straight-chain ($C_4$-$C_{10}$)-alkyl radical in which one $CH_2$ group can be replaced by O or S, |
| $A^1$and $A^2$ | independently of one another are 1,4-phenyl-one, 1,4-cyclohexylene or pyrimidine-2,5-diyl, |
| B | is CO-O or O-CO, |
| n1 | is 1, |
| n2 | is 0 or 1, |
| n3 | is 1 or 2 and |
| $R^1$ | is phenyl. |

**2.** A twistable liquid crystal phase containing at least one chiral compound, which comprises at least one chiral compound of the formula (I) as claimed in claim 1.

**3.** A liquid crystal display element containing a liquid crystal phase as claimed in claim 2.

**4.** The use of a chiral compound of the formula (I) as claimed in claim 1, for converting a tilted smectic liquid crystal phase into a ferroelectric liquid crystal phase.

**5.** A process for converting a tilted smectic liquid crystal phase into a ferroelectric liquid crystal phase by adding at least one chiral compound, which comprises adding at least one compound of the formula (I) as claimed in claim 1 to the liquid crystal phase.

**Claims for the following Contracting State : AT**

**1.** A process for preparing a chiral ester of the formula (I)

$$R^2-(A^1)_{n1}-(B)_{n2}-(A^2)_{n3}-O-CO-\underset{\underset{CF_3}{|}}{\overset{\overset{OCH_3}{|}}{C}}-R^1 \qquad (I)$$

in which the symbols are defined as follows:

| | |
|---|---|
| $R^2$ | is a straight-chain ($C_4$-$C_{10}$)-alkyl radical in which one $CH_2$ group can be replaced by O or S, |
| $A^1$and $A^2$ | independently of one another are 1,4-phenylene, 1,4-cyclohexylene or pyrimidine-2,5-diyl, |
| B | is CO-O or O-CO, |
| n1 | is 1, |
| n2 | is 0 or 1, |
| n3 | is 1 or 2 and |
| $R^1$ | is phenyl, |

which comprises reacting a mesogenic compound of the formula (III) or (III')

MOH (III)

$(MO)_mMe$ (III')

in which MO is as defined above, Me is an alkaline earth metal or alkali metal and m = 1 for Me = alkali metal and m = 2 for Me = alkaline earth metal, with a compound of the formula (IV)

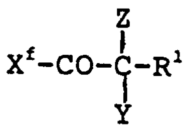

(IV)

in which $X^f$ is an OH group or halogen and Z = $OCH_3$, Y = $OCF_3$ and $R^1$ is as defined above.

**2.** The process as claimed in claim 1, wherein a mesogenic compound of the formula (III) or (III'), in which

| | |
|---|---|
| $R^2$ = | straight-chain ($C_4$-$C_{10}$)-alkyl, it being possible for one $CH_2$ group to be replaced by oxygen or sulfur, |
| $A^1$ and $A^2$ = | independently of one another unsubstituted 1,4-phenylene, 1,4-cyclohexylene or pyrimidine-2,5-diyl, |
| B = | CO-O or O-CO, |
| n1 = | 1, |
| n2 = | zero or 1 and |
| n3 = | 1 or 2, |

is reacted with a compound of the formula (IV), in which

| | |
|---|---|
| Y = | $CF_3$ and Z = $OCH_3$ or |
| Y = | $OCH_3$ and Z = H and |
| $R^1$ = | unsubstituted phenyl. |

**3.** A twistable liquid crystal phase containing at least one chiral compound, which comprises at least one chiral compound of the formula (I) as claimed in claim 1.

**4.** A liquid crystal display element containing a liquid crystal phase as claimed in claim 3.

**5.** The use of a chiral compound of the formula (I) as claimed in claim 1, for converting a tilted smectic liquid crystal phase into a ferroelectric liquid crystal phase.

**6.** A process for converting a tilted smectic liquid crystal phase into a ferroelectric liquid crystal phase by adding at least one chiral compound, which comprises adding at least one compound of the formula (I) as claimed in claim 1 to the liquid crystal phase.

**Claims for the following Contracting State : ES**

**1.** A process for preparing a chiral ester of the formula (I)

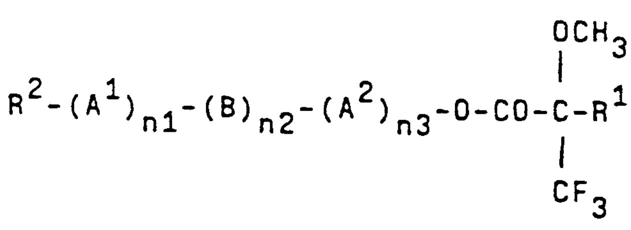

(I)

in which the symbols are defined as follows:

| | |
|---|---|
| $R^2$ | is a straight-chain ($C_4$-$C_{10}$)-alkyl radical in which one $CH_2$ group can be replaced by O or S, |
| $A^1$ and $A^2$ | independently of one another are 1,4-phenylene, 1-4-cyclohexylene or pyrimidine-2,5-diyl, |
| B | is CO-O or O-CO, |
| n1 | is 1, |
| n2 | is 0 or 1, |
| n3 | is 1 or 2 and |
| $R^1$ | is phenyl, |

which comprises reacting a mesogenic compound of the formula (III) or (III')

MOH (III)
$(MO)_mMe$ (III')

in which MO is as defined above, Me is an alkaline earth metal or alkali metal and m = 1 for Me = alkali metal and m = 2 for Me = alkaline earth metal, with a compound of the formula (IV)

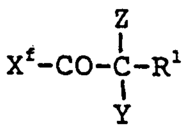

(IV)

in which $X^f$ is an OH group or halogen and Z = $OCH_3$, Y = $OCF_3$ and $R^1$ is as defined above.

**2.** The process as claimed in claim 1, wherein a mesogenic compound of the formula (III) or (III'), in which

| | |
|---|---|
| $R^2$ = | straight-chain ($C_4$-$C_{10}$)-alkyl, it being possible for one $CH_2$ group to be replaced by oxygen or sulfur, |
| $A^1$ and $A^2$ = | independently of one another unsubstituted 1,4-phenylene, 1,4-cyclohexylene or pyrimidine-2,5-diyl, |
| B = | CO-O or O-CO, |
| n1 = | 1, |
| n2 = | zero or 1 and |
| n3 = | 1 or 2, |

is reacted with a compound of the formula (IV), in which

| | |
|---|---|
| Y = | $CF_3$ and Z = $OCH_3$ or |
| Y = | $OCH_3$ and Z = H and |
| $R^1$ = | unsubstituted phenyl. |

**3.** A process for converting a tilted smectic liquid crystal phase into a ferroelectric liquid crystal phase by adding at least one chiral compound, which comprises adding at least one compound of the formula (I) as claimed in claim 1 to the liquid crystal phase.

**4.** A liquid crystal display element containing a liquid crystal phase as claimed in claim 3.

## Revendications

**Revendications pour les Etats contractants suivants : BE, CH, DE, FR, GB, IT, LI, LU, NL, SE**

**1.** Esters chiraux de formule I

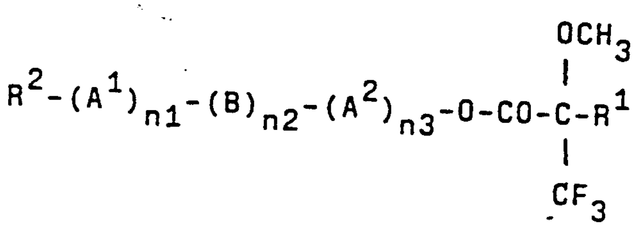

(I)

dans laquelle

$R^2$ est un radical alkyle en $C_4$-$C_{10}$ à chaîne droite, dans lequel un groupe $CH_2$ peut être remplacé par O ou S,

$A^1$ et $A^2$, indépendamment l'un de l'autre, sont chacun un radical phénylène-1,4, cyclohexylène-1,4 ou pyrimidine-diyle-2,5,

B est CO-O ou O-CO,

n1 vaut 1,

n2 vaut 0 ou 1,

n3 vaut 1 ou 2 et
$R^1$ est le radical phényle.

2. Phase mésomorphe torsadable contenant au moins un composé chiral, caractérisée en ce qu'elle contient au moins un composé chiral ayant la formule générale (I) selon la revendication 1.

3. Elément d'affichage à cristaux liquides contenant une phase mésomorphe selon la revendication 3.

4. Utilisation d'un composé chiral de formule générale (I) selon la revendication 1 pour la conversion d'une phase mésomorphe inclinée-smectique en une phase mésomorphe ferro-électrique.

5. Procédé de conversion d'une phase mésomorphe inclinée smectique en une phase mésomorphe ferro-électrique par addition d'au moins un composé chiral, caractérisé en ce qu'on ajoute à la phase mésomorphe au moins un composé de formule générale (I) selon la revendication 1.

**Revendications pour l'Etat contractant suivant : AT**

1. Procédé de préparation d'esters chiraux de formule (I)

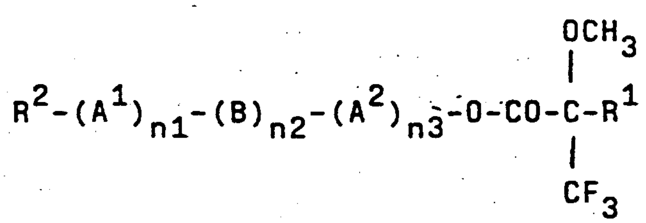
(I)

dans laquelle :
$R^2$ est un radical alkyle en $C_4$-$C_{10}$ à chaîne droite, dans lequel un groupe $CH_2$ peut être remplacé par O ou S,
$A^1$ et $A^2$, indépendamment l'un de l'autre, sont chacun un radical phénylène-1,4, cyclohexylène-1,4 ou pyrimidine-diyle-2,5,
B est CO-O ou O-CO,
n1 vaut 1,
n2 vaut 0 ou 1,
n3 vaut 1 ou 2 et
$R^1$ est le radical phényle
caractérisé en ce qu'on fait réagir des composés mésogènes de formules (III) ou (III')

MOH (III)
$(MO)_m$Me (III')

dans lesquelles MO a la signification donnée ci-dessus, Me représente un métal alcalino-terreux ou de préférence un métal alcalin, et m = 1 (Me est un métal alcalin) ou m = 2 (Me est un métal alcalino-terreux), avec des composés de formule (IV)

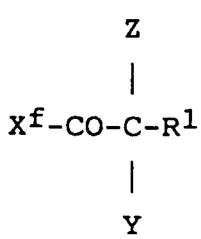
(IV)

dans laquelle $X^f$ est un groupe OH ou un halogène, avec Z = $OCH_3$, Y = $OCF_3$, $R^1$ ayant les

significations données ci-dessus.

**2.** Procédé selon la revendication 1, caractérisé en ce qu'on fait réagir des composés mésogènes de formules (III) ou (III') dans lesquelles :

$R^2$ est un radical alkyle en $C_4$-$C_{10}$ à chaîne droite, dans lequel un groupe $CH_2$ peut être remplacé par O ou S,

$A^1$ et $A^2$, indépendamment l'un de l'autre, sont chacun un radical phénylène-1,4, cyclohexylène-1,4 ou pyrimidine-diyle-2,5,

B est CO-O ou O-CO,

n1 vaut 1,

n2 vaut 0 ou 1,

n3 vaut 1 ou 2 et

avec un composé de formule (IV) dans laquelle Y = $CF_3$ et Z = $OCH_3$, ou bien Y = $OCH_3$ et Z = H, et $R^1$ est un radical phényle non substitué.

**3.** Phase mésomorphe torsadable contenant au moins un composé chiral, caractérisée en ce qu'elle contient au moins un composé chiral ayant la formule générale (I) selon la revendication 1.

**4.** Elément d'affichage à cristaux liquides contenant une phase mésomorphe selon la revendication 3.

**5.** Utilisation d'un composé chiral de formule générale (I) selon la revendication 1 pour la conversion d'une phase mésomorphe inclinée-smectique en une phase mésomorphe ferro-électrique.

**6.** Procédé de conversion d'une phase mésomorphe inclinée smectique en une phase mésomorphe ferro-électrique par addition d'au moins un composé chiral, caractérisé en ce qu'on ajoute à la phase mésomorphe au moins un composé de formule générale (I) selon la revendication 1.

**Revendications pour l'Etat contractant suivant : ES**

**1.** Procédé de préparation d'esters chiraux de formule (I)

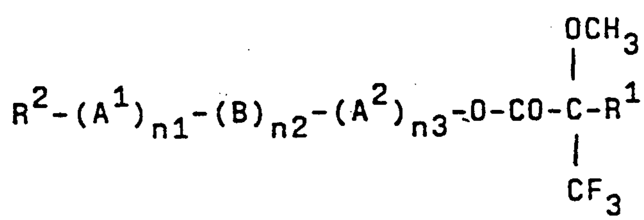

(I)

dans laquelle :

$R^2$ est un radical alkyle en $C_4$-$C_{10}$ à chaîne droite, dans lequel un groupe $CH_2$ peut être remplacé par O ou S,

$A^1$ et $A^2$, indépendamment l'un de l'autre, sont chacun un radical phénylène-1,4, cyclohexylène-1,4 ou pyrimidine-diyle-2,5,

B est CO-O ou O-CO,

n1 vaut 1,

n2 vaut 0 ou 1,

n3 vaut 1 ou 2 et

$R^1$ est le radical phényle

caractérisé en ce qu'on fait réagir des composés mésogènes de formules (III) ou (III')

MOH (III)

$(MO)_mMe$ (III')

dans lesquelles MO a la signification donnée ci-dessus, Me représente un métal alcalino-terreux ou de préférence un métal alcalin, et m = 1 (Me est un métal alcalin) ou m = 2 (Me est un métal alcalino-terreux), avec des composés de formule (IV)

$$\begin{array}{c} Z \\ | \\ X^{f}\text{-CO-C-}R^{1} \\ | \\ Y \end{array} \qquad (IV)$$

dans laquelle $X^f$ est un groupe OH ou un halogène, avec Z = $OCH_3$, Y = $OCF_3$, $R^1$ ayant les significations données ci-dessus.

**2.** Procédé selon la revendication 1, caractérisé en ce qu'on fait réagir des composés mésogènes de formules (III) ou (III') dans lesquelles :

$R^2$ est un radical alkyle en $C_4$-$C_{10}$ à chaîne droite, dans lequel un groupe $CH_2$ peut être remplacé par O ou S,

$A^1$ et $A^2$, indépendamment l'un de l'autre, sont chacun un radical phénylène-1,4, cyclohexylène-1,4 ou pyrimidine-diyle-2,5,

B est CO-O ou O-CO,

n1 vaut 1,

n2 vaut 0 ou 1,

n3 vaut 1 ou 2 et

avec un composé de formule (IV) dans laquelle Y = $CF_3$ et Z = $OCH_3$, ou bien Y = $OCH_3$ et Z = H, et $R^1$ est un radical phényle non substitué.

**3.** Procédé de conversion d'une phase mésomorphe inclinée smectique en une phase mésomorphe ferroélectrique par addition d'au moins un composé chiral, caractérisé en ce qu'on ajoute à la phase mésomorphe au moins un composé de formule générale (I) selon la revendication 1.

**4.** Elément d'affichage à cristaux liquides contenant une phase mésomorphe selon la revendication 3.